# EUROPÄISCHE PATENTSCHRIFT

(11) **EP 1 597 247 B1**
(45) Veröffentlichungstag und Bekanntmachung des Hinweises auf die Patenterteilung: **23.07.2008**
(21) Anmeldenummer: 04707896.9
(22) Anmeldetag: 04.02.2004
(51) Int. Cl.: C07D 333/36, C07D 333/38, C07D 333/40, C07D 333/44, C07D 413/04, C07D 409/04, A61K 31/38, A61K 31/41, A61K 31/445, A61P 3/10

(54) **SUBSTITUIERTE 3-(BENZOYLUREIDO)-THIOPHENDERIVATE, VERFAHREN ZU DEREN HERSTELLUNG UND DEREN VERWENDUNG**
SUBSTITUTED 3-(BENZOYLUREIDO)-THIOPHENE DERIVATIVES, METHOD FOR THE PRODUCTION AND USE THEREOF
DERIVES DE 3-(BENZOYLUREIDO)-THIOPHENE SUBSTITUES, PROCEDE DE FABRICATION ASSOCIE ET LEUR UTILISATION

(30) Priorität: 17.02.2003 DE 10306502
(43) Veröffentlichungstag der Anmeldung: 23.11.2005
(73) Patentinhaber: Sanofi-Aventis Deutschland GmbH, 65929 Frankfurt am Main (DE)
(72) Erfinder: SCHOENAFINGER, Karl, 63755 Alzenau (DE); DEFOSSA, Elisabeth, 65510 Idstein (DE); VON ROEDERN, Erich, 65795 Hattersheim (DE); KADEREIT, Dieter, D-65926 Frankfurt am Main (DE); HERLING, Andreas, 65520 Bad Camberg (DE); BURGER, Hans-Joerg, Morristown, NJ 07960 (US); KLABUNDE, Thomas, 65929 Frankfurt (DE); WENDT, Karl-Ulrich, D-65926 Frankfurt am Main (DE)
(86) Internationale Anmeldenummer: PCT/EP2004/000993
(87) Internationale Veröffentlichungsnummer: WO 2004/072060

(56) Entgegenhaltungen:
- EP-A- 0 047 496
- EP-A- 0 300 972
- US-A1- 2002 151 586

## Beschreibung

Die Erfindung betrifft substituierte 3-(Benzoylureido)-thiophenderivate sowie deren physiologisch verträgliche Salze und physiologisch funktionelle Derivate.

In EP 0 300 972 sind Benzoylureido-Thiophene mit pestizider, speziell insektizider und akarizider Wirkung beschrieben.

In US2002/0151586 sind Benzoylureido-Benzene zur Behandlung von Diabetes beschrieben.

Der Erfindung lag die Aufgabe zugrunde, Verbindungen zur Verfügung zu stellen, mit denen eine Prävention und Behandlung von Diabetes Mellitus möglich ist. Die Verbindungen sollten dazu insbesonders eine therapeutisch verwertbare Blutzucker senkende Wirkung entfalten.

Die Erfindung betrifft daher Verbindungen der Formel I, 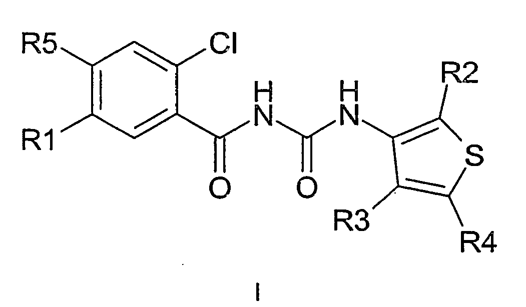 worin bedeuten
- R5: F, Cl oder Br;
- R1: H, F, Cl, Br;
- R2: H, F, Cl, Br, (C₁-C₆)-Alkyl, CF₃, OCF₃, NO₂, CN, O-(C₁-C₆)-Alkyl, CO(C₁-C₆)-Alkyl, COOH, COO(C₁-C₆)-Alkyl, CONH₂, CONH(C₁-C₆)-Alkyl, CON((C₁-C₆)-Alkyl)₂, SO₂-(C₁-C₆)-Alkyl, oder den Rest A;
- R3: H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- R4: H, (C₁-C₆)-Alkyl, COO(C₁-C₆)-Alkyl, SO₂-(C₁-C₆)-Alkyl, SO₂-Pipridinyl, SO₂-Piperazinyl, (C₁-C₆)-Alkyl-Phenyl, wobei der Phenylring, der Piperidinylring und der Piperazinylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- A: einen heterozyklischen Rest der Formel 2a, 2b, 2c oder 3; 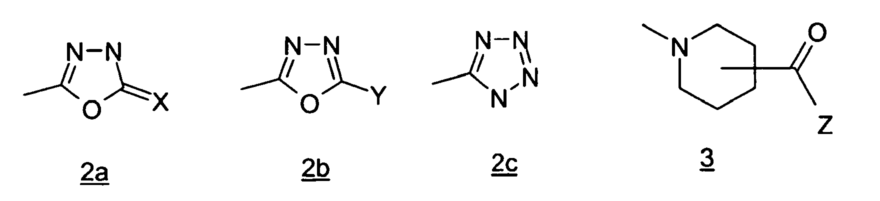
- X: O oder NH;
- Y: OH oder NH₂;
- Z: OH, O(C₁-C₆)-Alkyl, NH₂, NH(C₁-C₆)-Alkyl, N((C₁-C₆)-Alkyl)₂;
sowie deren physiologisch verträgliche Salze.

Bevorzugt sind Verbindungen der Formel I worin ein oder mehrere Reste folgende Bedeutung haben:
- R5: F, Cl oder Br;
- R1: H, F;
- R2: H, F, Cl, Br, (C₁-C₆)-Alkyl, CF₃, OCF₃, NO₂, CN, O-(C₁-C₆)-Alkyl, CO(C₁-C₆)-Alkyl, COOH, COO(C₁-C₆)-Alkyl, CONH₂, CONH(C₁-C₆)-Alkyl, CON((C₁-C₆)-Alkyl)₂, SO₂-(C₁-C₆)-Alkyl, oder den Rest A;
- R3: H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F oder Cl substituiert sein kann;
- R4: H, (C₁-C₆)-Alkyl, COO(C₁-C₆)-Alkyl, SO₂-(C₁-C₆)-Alkyl, SO₂-Pipridinyl, SO₂-Piperazinyl, (C₁-C₆)-Alkyl-Phenyl, wobei der Phenylring, der Piperidinylring und der Piperazinylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- A: einen heterozyklischen Rest der Formel 2a, 2b, 2c oder 3; 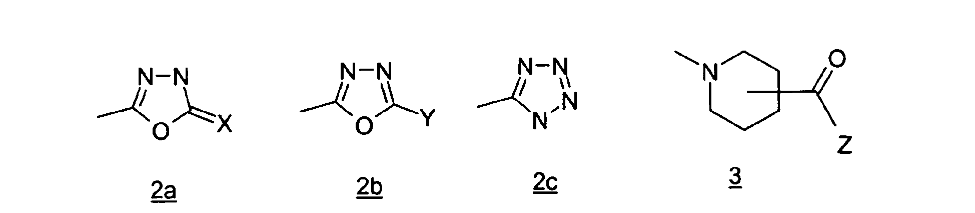
- X: O oder NH;
- Y: OH oder NH₂;
- Z: OH;
sowie deren physiologisch verträgliche Salze.

Besonders bevorzugt sind Verbindungen der Formel I worin ein oder mehrere Reste folgende Bedeutung haben:
- R5: F;
- R1: F;
- R2: COOH, COO(C₁-C₆)-Alkyl, CONH₂, CONH(C₁-C₆)-Alkyl, CON((C₁-C₆)-Alkyl)₂, oder den Rest A;
- R3: H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F substituiert sein kann;
- R4: H, (C₁-C₆)-Alkyl, COO(C₁-C₆)-Alkyl, SO₂-(C₁-C₆)-Alkyl, SO₂-Pipridinyl, SO₂-Piperazinyl, (C₁-C₆)-Alkyl-Phenyl, wobei der Phenylring, der Piperidinylring und der Piperazinylring bis zu zweifach mit F oder (C₁-C₆)-Alkyl, substituiert sein kann;
- A: einen heterozyklischen Rest der Formel 2a oder 2b; 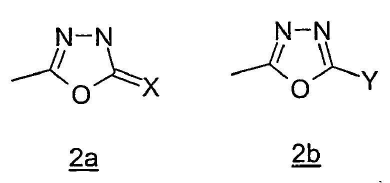
- X: O oder NH;
- Y: OH oder NH₂;
sowie deren physiologisch verträgliche Salze.

Weiterhin bevorzugt sind Verbindungen der Formel I, worin mindestens einer der Reste R2, R3 und R4 ungleich Wasserstoff ist.

Die Erfindung bezieht sich auf Verbindungen der Formel I, in Form ihrer Razemate, razemischen Mischungen und reinen Enantiomere sowie auf ihre Diastereomere und Mischungen davon.

Die Alkylreste in den Substituenten R2, R3 und R4, können sowohl geradkettig wie verzweigt sein.

Können Reste oder Substituenten mehrfach in den Verbindungen der Formel. I auftreten, wie zum Beispiel A, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Arylrest wird ein Phenyl-, Naphthyl-, Biphenyl-, Tetrahydronaphthyl-, alpha- oder beta-Tetralon-, Indanyl- oder Indan-1-on-ylrest verstanden.

Die hier verwendeten Begriffe "Heterocyclischer Ring" bzw. "Heterocyclischer Rest" beziehen sich auf Heteroarylreste und Heterocycloalkylrerste, die sich aus 3 bis 10 glieddrigen Kohlenstoffringen ableiten, in denen ein oder mehrere Kohlenstoffatome durch ein oder mehrere Atome, ausgewählt aus der Gruppe Sauerstoff, Schwefel und Stickstoff, ersetzt sind.

Geeignete "Heterocyclische Ringe" bzw. "Heterocyclischer Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Verbindung(en) der Formel (I) können auch in Kombination mit weiteren Wirkstoff verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise Von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel 1. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale- (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine ÖI-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiveh/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel 1 mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart. Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe. Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel 1 in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) Antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem a-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid. Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methy)-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel 1 und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

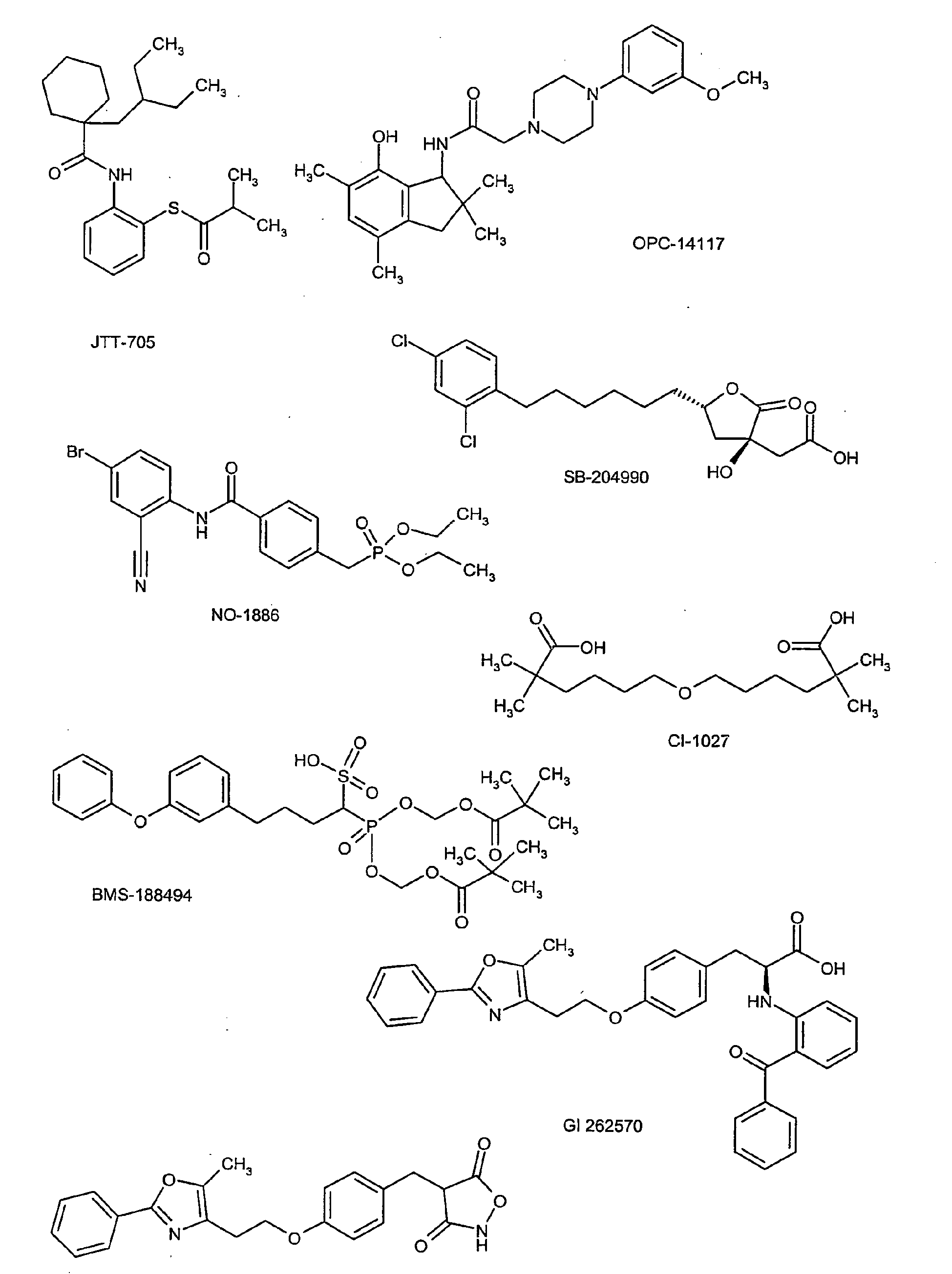

Die nachfolgend aufgeführten Beispiele dienen zur Erläuterung der Erfindung, ohne diese jedoch einzuschränken.

### Analog wurden die Verbindungen der folgenden Beispiele hergestellt:

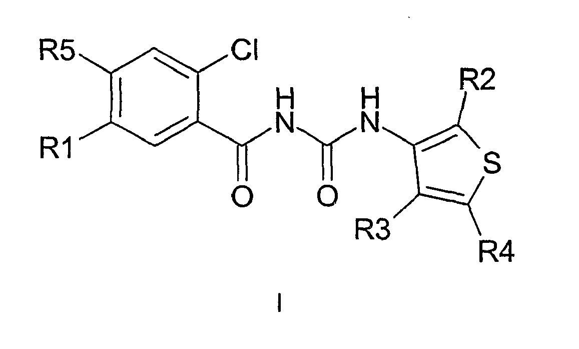

| **Bsp.** | **R5** | **R1** | **R2** | **R3** | **R4** | **Fp.** |
|---|---|---|---|---|---|---|
| 1d | F | F | 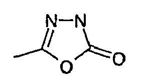 | H | H | 236,1 |
| 2c | F | F | 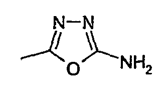 | H | H | Harz |
| 3b | F | F | COOH | H | 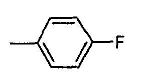 | 227,7 |
| 4d | F | F | 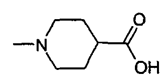 | H | 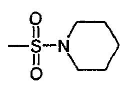 | 219,9 |
| 5 | F | F | COOMe | H | H | |
| 6 | F | F | CONH₂ | H | H | |
| 7 | F | F | COOMe | H | SO₂Me | |
| 8 | F | F | COOMe | H | 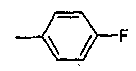 | |
| 9 | F | F | COOMe | Me | H | |
| 10 | F | F | H | COOMe | H | |
| 11 | F | F | NO₂ | H | H | |
| 12 | F | F | COOH | SO₂Ph | H | 193,5 |
| 13 | F | F | COOH | SO₂iPr | H | 164,8 |
| 14 | F | F | COOMe | H | Ph | 208,6 |
| 15 | F | F | COMe | H | Ph | 204,8 |
| 16 | F | F | CONH₂ | H | tert.Butyl | >300 |
| 17 | F | F | CONH₂ | H | Ph | >300 |
| 18 | F | F | CONH₂ | H | 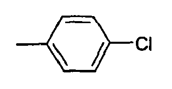 | >300 |
| 19 | F | F | 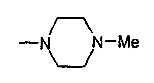 | H | 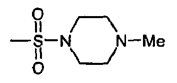 | 225,8 |
| 20 | F | F | 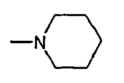 | H | 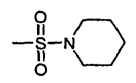 | 188,1 |
| 21 | F | F | COOH | H | Ph | 226,5 |
| 22 | F | F | CONH₂ | H | 3-Thienyl | >300(Zers.) |
| 23 | F | F | CONH₂ | H | 2-Thienyl | >250(Zers.) |
| 24 | F | F | CONH₂ | H | 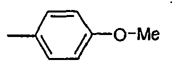 | >300 |
| 25 | F | F | -CN | H | 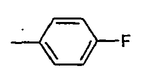 | >200(Zers) |
| 26 | F | F | COMe | H | H | 180(Zers.) |
| 27 | F | F | CONH₂ | H | 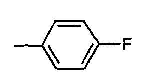 | 237,2 |
| 28 | F | F | H | 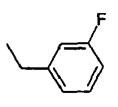 | H | |
| 29 | F | F | H | COOMe | COOMe | |
| 30 | F | F | H | Ph | H | |
| 31 | F | F | H | F | H | |
| 32 | F | F | H | Me | H | |
| 33 | F | H | 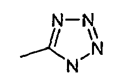 | H | H | 219,8 |
| 34 | F | F | H | H | Phenyl | 178,7 |
| 35 | F | F | COOH | H | H | 214,8 |
| 36c | F | F | 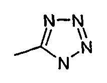 | H | 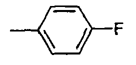 | 206,2 |
| 37 | F | H | 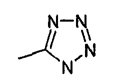 | H | 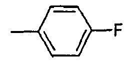 | 217,8 |

### Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde. Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben. Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| **Bsp.** | **IC-50 (µM)** |
|---|---|
| 1 d | 0,03 |
| 2c | 0,45 |
| 3b | 0,01 |
| 4d | 0,11 |
| 6 | 0,70 |
| 15 | 0,17 |
| 17 | 0,03 |
| 21 | 0,01 |
| 23 | 0,05 |
| 24 | 0,04 |
| 30 | 1,40 |
| 33 | 0,08 |

Aus der Tabelle ist abzulesen, dass die Verbindungen der Formel I die Aktivität der Glykogenphosphorylase a hemmen und dadurch zur Senkung des Blutzuckerspiegels gut geeignet sind.

Nachfolgend wird die Herstellung einiger Beispiele detailliert beschrieben, die übrigen Verbindungen der Formel I wurden analog erhalten:
Experimenteller Teil:

### Beispiel 1:

### a) 3-(tert.Butoxicarbonylamino)-thiophen-2-carbonsäure-hydrazid

Zur Lösung von 1,3 g 3-(tert.Butoxicarbonylamino)-thiophen-2-carbonsäure-methylester in 10 ml Ethanol werden 0,4 g Hydrazinhydrat gegeben und die Mischung 5 Stunden am Rückfluß gekocht. Nach dem Entfernen der flüchtigen Anteile im Vakuum bei 40°C wurde das verbleibende Öl durch Säulenchromatographie (Kieselgel, Laufmittel: Methylenchlorid: Methanol = 95:5) gereinigt.
Ausbeute. 740 mg Fp.: 146,5°C

### b) [2-(5-Oxo-4,5-dihydro-[1,3,4]oxdiazol-2-yl)-thiophen-3-yl]-carbamnsäure-tert-butyl-ester

Zur Lösung von 240 mg 3-(tert.Butoxicarbonylamino)-thiophen-2-carbonsäure-hydrazid in 5 ml THF werden 4 ml einer 20%igen toluolischen Phosgenlösung getropft und die Mischung bei RT gerührt. Nach 1 Stunde wird mit 10 ml Wasser versetzt und nach kurzem Digerieren mit Essigester extrahiert. Nach dm Trocknen der Essigesterphase mit Natriumsulfat wird im Vakuum eingeengt und der verbleibende Rückstand ohne weitere Reinigung weiter verwendet.
Ausbeute: 120 mg Fp.: 180°C

### c) 5-(3-Amino-thiophen-2-yl)-3H-[1,3,4]oxdiazol-2-on-hydrochlorid

Die Mischung bestehend aus 100 mg [2-(5-Oxo-4,5-dihydro-[1,3,4]oxdiazol-2-yl)-thiophen-3-yl]-carbaminsäure-tert-butyl-ester und 5 ml einer 4 molaren HCL-Lösung in Dioxan wird eine Stunde gerührt. Danach werden die flüchtigen Anteil im Vakuum abgezogen und der Rückstand mit 5ml tert.Butylmethylether verrührt und das Produkt abgesaugt und im Vakuum getrocknet.
Ausbeute: 60 mg Fp.: 211°C

### d) 1-(2-Chlor-4,5-difluor-benzoyl)-3-[2-(5-oxo-4,5-dihydro-[1,3,4]oxdiazol-2-yl)-thiophen-3-yl]-harnstoff

5-(3-Amino-thiophen-2-yl)-3H-[1,3,4]oxdiazol-2-on-hydröchiorid (30 mg) wird in 3 ml Acetonitril vorgelegt. Die äquimolare Lösung von 2-Chlor-4,5-difluor-benzoyl-isocyanat in Acetonitril wird zugefügt. Nach 3 Stunden wird der Feststoff abgesaugt und im Vakuum getrocknet.
Ausbeute: 50 mg Fp.: 236,1°C

### Beispiel 2:

### a) 2-(5-Amino-[1,3,4]oxdiazol-2-yl)-thiophen-3-yl]-carbaminsäure-tert-butyl-ester

Die Lösung von 192 mg 3-(tert.Butoxicarbonylamino)-thiophen-2-carbonsäure-hydrazid in 4 ml Acetonitril wird mit 0,17 ml einer 5 molaren Bromcyanlösung in Acetonitril und 60 mg Pottasche versetzt. Die Mischung wird 4 Stunden bei RT gerührt, der Feststoff abfiltriert und das Filtrat im Vakuum eingeengt. Das gewünschte Produkt wird dann säulenchromatografisch (Kiedelgel, Laufmittel: Methylenchlorid: Methanol = 95:5) gereinigt.
Ausbeute: 90 mg Fp.: Harz

### b) 5-(3-Amino-thiophen-2-yl)-[1,3,4]oxdiazol-2-ylamine-hydrochlorid

2-(5-Amino-[1,3,4]oxdiazol-2-yl)-thiophen-3-yl]-carbaminsäure-tert-butyl-ester (90 mg) werden in 5 ml 4 molare HCl-Lösung in Dioxan gegeben und das Gemisch 1 stunde bei RT gerührt. Nach dem Einengen im Vakuum wird der Rückstand mit tert.Butylmethylether verrührt und der Feststoff abgesaugt und im Vakuum getrocknet.
Ausbeute: 60 mg Fp.: >250° (Zers.)

### c) 1-[2-(5-Amino-[1,3,4]oxdiazol-2-yl)-thiophen-3-yl]-3-(2-chloro-4,5-difluoro-benzoyl)-harnstoff

5-(3-Amino-thiophen-2-yl)-[1,3,4]oxdiazol-2-ylamine-hydrochlorid (30 mg) wird in 3 ml Acetonitril vorgelegt. Die äquimolare Lösung von 2-Chlor-4,5-difluor-benzoyl-isocyanat in Acetonitril wird zugefügt und bei RT gerührt. Nach 3 Stunden wird der Feststoff abgesaugt und im Vakuum getrocknet.
Ausbeute: 25 mg Fp.: Harz

### Beispiel 3:

### a) 3-Amino-5-(4-fluor-phenyl)-thiophen-2-carbonsäure

Die Mischung bestehend aus 500 mg 3-Amino-5-(4-fluor-phenyl)-thiophen-2-carbonsäure-methylester 160 mg Lithiumhydroxid, 2 ml Wasser, 2 ml THF und 2 ml Methanol wird 3 Tage lang bei RT gerührt. Nach dem Verdünnen mit 15 ml Wasser wird unverseifter Ester durch Ausschütteln mit Essigester entfernt. Die wässrige Phase wird mit Salzsäure auf pH = 5 gestellt, verrührt und der ausgefallene Niederschlag abgesaugt und getrocknet.
Ausbeute: 260 mg Fp.: 145,2°C (roh)

### b) 3-[3-(2-Chlor-4,5-difluor-benzoyl)-ureido]-5-(4-fluor-phenyl)-thiophen-2-carbonsäure

3-Amino-5-(4-fluor-phenyl)-thiophen-2-carbonsäure (50 mg) wird in 3 ml Acetonitril vorgelegt. Die äquimolare Lösung von 2-Chlor-4,5-difluor-benzoyl-isocyanat in Acetonitril wird zugefügt und die Mischung bei RT gerührt. Nach 3 Stunden wird der Feststoff abgesaugt nochmals mit Methanol verrührt, abgesaugt und im Vakuum getrocknet.
Ausbeute: 46 mg Fp.: 227,7°C

### Beispiel 4:

### a) 1-(5-Chlor-4-nitro-thiophen-2-sulfonyl)-piperidin und 1-(5-Piperidino-4-nitro-thiophen-2-sulfonyl)-piperidin

Zur Lösung von 2,6 g 5-Chlor-4-nitro-thiophen-2-sulfonylchlorid in 8 ml NMP werden unter Umrühren und Eiskühlung 1,7 g Piperidin getropft. Es wird noch 30 Minuten bei RT gerührt, mit 30 ml Wasser verdünnt und der sich bildende Niederschlag nach dem Verrühren abgesaugt. Die beiden Produkte werden säulenchromatografisch (I<ieselgel, LM: Essigester:nHeptan = 1:1)getrennt. 1-(5-Piperidino-4-nitro-thiophene-2-sulfonyl)-piperidin: Ausbeute: 1,3g Fp.: 151,3°C 1-(5-Chlor-4-nitro-thiophen-2-sulfonyl)-piperidin: Ausbeute.: 0,85g Fp.: 136,4°C

### b) 1-(3-Nitro-5-piperidin-1-sulfonyl-thiophen-2-yl)-piperidin-4-carbonsäure

Die Mischung aus 310 mg 1-(5-Chlor-4-nitro-thiophen-2-sulfonyl)-piperidin, 250 mg Piperidin-4-carbonsäure und_3 ml NMP wird 1 h bei 85 °C gerührt, abgekühlt, mit 15 ml Wasser verdünnt und verrührt. Der Niederschlag wird abgesaugt, mit Wasser gewaschen und aus Isopropanol/Wasser(4:1) umkristallisiert.
Ausbeute: 310 mg Fp.: 165,8°C

### c) 1-[3-Amino-5-(piperidin-1-sulfonyl)-thiophen-2-yl]-piperidin-4-carbonsäure

Die Lösung von 300 mg 1-(3-Nitro-5-piperidin-1-sulfonyl-thiophen-2-yl)-piperidin-4-carbonsäure in 20 ml Essigester wird mit 1,0 g Zinn-2-chlorid versetzt und 8 h bei 65 °C gerührt. Nach dem Abkühlen wird mit 30 ml Wasser verrührt, über Cellite abgesaugt und die Essigesterphase abgetrennt, getrocknet und im Vakuum eingeengt. Dieses Rohprodukt wurde ohne weitere Reinigung weiterverwendet.
Ausbeute: 320 mg (roh) Fp.: Harz

### c) 1-[3-[3-(2-Chlor-4,5-difluor-benzoyl)-ureido]-5-(piperidin-1-sulfonyl)-thiophen-2-yl]- piperidin-4-carbonsäure

1-[3-Amino-5-(piperidin-1-sulfonyl)-thiophen-2-yl]-piperidin-4-carbonsäure (0,2g Rohprodukt) wird in2 ml Acetonitril gelöst, mit der äquimolaren Lösung von 2-Chlor-4,5-difluor-benzoyl-isocyanat in Acetonitril versetzt und die Mischung bei RT gerührt. Nach 3 Stunden wird der Feststoff abgesaugt und im Vakuum getrocknet.
Ausbeute: 85 mg Fp.: 219,9°C

### Beispiel 36:

### a) 5-(4-Fluor-phenyl)-2-(1 H-tetrazol-5-yl)-thiophen-3-ylamin

Die Mischung bestehend aus 0,65 g 3-Amino-2-cyano-5-(4-fluorphenyl)-thiophen, 7,5 ml Xylol und 0,93 g Trimethylzinnazid wird drei Stunden bei 130-140°C gerührt. Danach wird die Mischung im Vakuum bei 40°C eingeengt und der Rückstand mit Wasser unter Zusatz von 1 % Trifluoressigsäure verrührt. Der Niederschlag wird abgesaugt und im Vakuum bei 40°C getrocknet.
Ausbeute: 0,6 g Fp.: 217,3°C

### c) 1-[4-(4-Fluorphenyl-2-(tetrazol-5-yl)-thiophen-3-yl]-3-(2-chloro-4,5-difluoro-benzoyl)-harnstoff

5-(4-Fluor-phenyl)-2-(1 H-tetrazol-5-yl)-thiophen-3-ylamin (0,1 g) wird in 2 ml Acetonitril gelöst, mit der äquimolaren Lösung von 2-Chlor-4,5-difluor-benzoyl-isocyanat in Acetonitril versetzt und die Mischung bei RT gerührt. Nach 3 Stunden wird der Feststoff abgesaugt und im Vakuum getrocknet.
Ausbeute: 73 mg Fp.: 206,2°C

Die Verbindungen der Formel I können hergestellt werden, dadurch dass Harnstoffe der Formel 5 oder 3-Aminothiophenderivate der Formel 6, mit Benzoesäurederivaten der Formel 4, 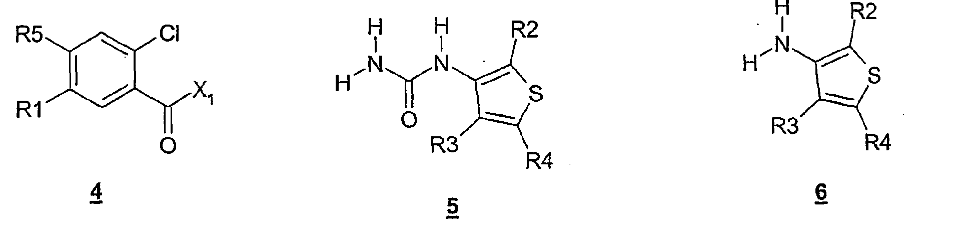 worin R1 bis R5 die oben angegebenen Bedeutungen haben und X1 gleich Cl oder NCO sein kann, mit Säurechloriden oder Anhydriden bzw. Benzoylisocyanaten, umgesetzt werden.

## Patentansprüche

1. Verbindungen der Formel I, 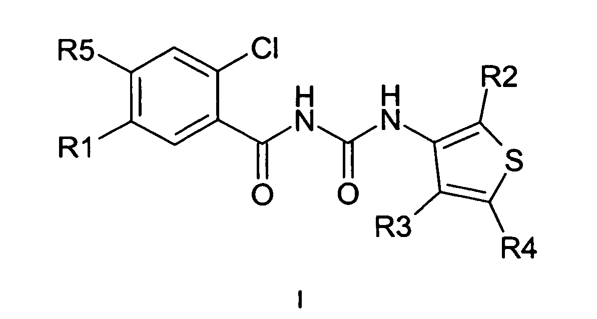 worin bedeuten
R5 F, Cl oder Br;
R1 H, F, Cl, Br;
R2 H, F, Cl, Br, (C₁-C₆)-Alkyl, CF₃, OCF₃, NO₂, CN, O-(C₁-C₆)-Alkyl, CO(C₁-C₆)-Alkyl, COOH, COO(C₁-C₆)-Alkyl, CONH₂, CONH(C₁-C₆)-Alkyl, CON((C₁-C₆)-Alkyl)₂, SO₂-(C₁-C₆)-Alkyl, oder den Rest A;
R3 H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
R4 H, (C₁-C₆)-Alkyl, COO(C₁-C₆)-Alkyl, SO₂-(C₁-C₆)-Alkyl, SO₂-Piperidinyl, SO₂-Piperazinyl (C₁-C₆)-Alkyl-Phenyl, wobei der Phenylring, der Piperidinylring und der Piperazinylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
A einen heterozyklischen Rest der Formel 2a, 2b, 2c oder 3; 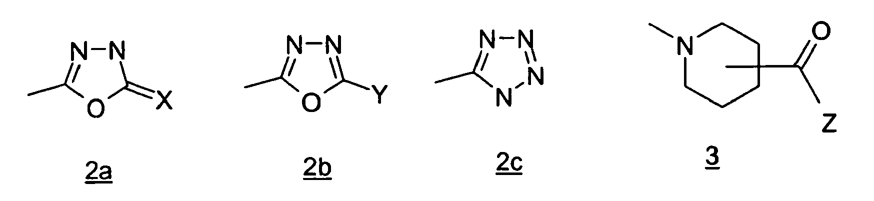
X O oder NH;
Y OH oder NH₂;
Z OH, O(C₁-C₆)-Alkyl, NH₂, NH(C₁-C₆)-Alkyl, N((C₁-C₆)-Alkyl)₂;
oder Verbindungen der Formel I, worin bedeuten 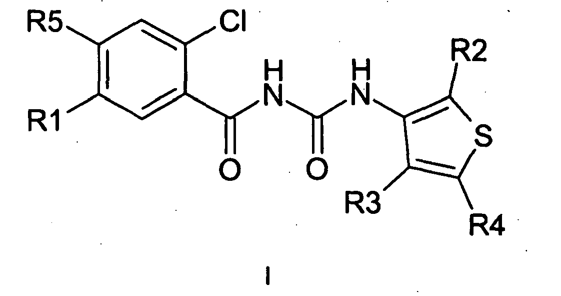
| **R5** | **R1** | **R2** | **R3** | **R4** |
|---|---|---|---|---|
| F | F | COOH | H | 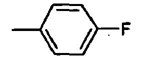 |
| F | F | COOMe | H | 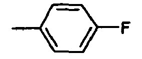 |
| F | F | COOMe | H | Ph |
| F | F | COMe | H | Ph |
| F | F | CONH₂ | H | Ph |
| F | F | CONH₂ | H | 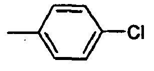 |
| F | F | COOH | H | Ph |
| F | F | CONH₂ | H | 3-Thienyl |
| F | F | CONH₂ | H | 2-Thienyl |
| F | F | CONH₂ | H | 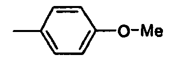 |
| F | F | -CN | H | 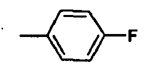 |
| F | F | CONH₂ | H | 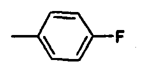 |
| F | F | H | H | Phenyl |
| F | F | 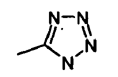 | H | 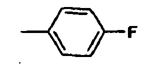 |
| F | H | 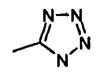 | H | 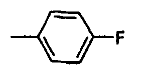 |
sowie deren physiologisch verträgliche Salze.

2. Verbindungen der Formel I, gemäß Anspruch 1, **dadurch gekennzeichnet, daß** darin bedeuten
R5 F, Cl oder Br;
R1 H, F;
R2 H, F, Cl, Br, (C₁-C₆)-Alkyl, CF₃, OCF₃, NO₂, CN, O-(C₁-C₆)-Alkyl, CO(C₁-C₆)-Alkyl, COOH, COO(C₁-C₆)-Alkyl, CONH₂, CONH(C₁-C₆)-Alkyl, CON((C₁-C₆)-Alkyl)₂, SO₂-(C₁-C₆)-Alkyl, oder den Rest A;
R3 H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F oder Cl substituiert sein kann;
R4 H, (C₁-C₆)-Alkyl, COO(C₁-C₆)-Alkyl, SO₂-(C₁-C₆)-Alkyl, SO₂-Piperidinyl,
SO₂-Piperazinyl, (C₁-C₆)-Alkyl-Phenyl, wobei der Phenylring, der Piperidinylring und der Piperazinylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
A einen heterozyklischen Rest der Formel 2a, 2b, 2c oder 3; 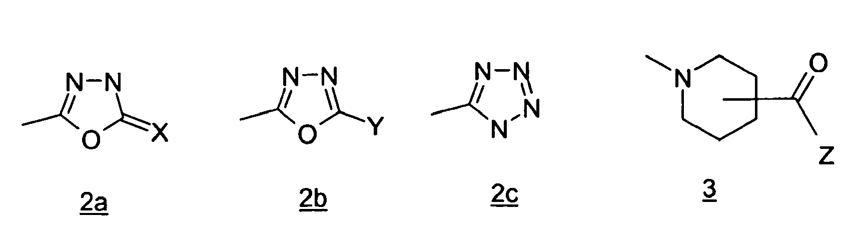
X O oder NH;
Y OH oder NH₂;
Z OH;
sowie deren physiologisch verträgliche Salze.

3. Verbindungen der Formel I, gemäß Anspruch 1 oder 2, **dadurch gekennzeichnet, daß** darin bedeuten
R5 F;
R1 F;
R2 COOH, COO(C₁-C₆)-Alkyl, CONH₂, CONH(C₁-C₆)-Alkyl, CON((C₁-C₆)-Alkyl)₂, oder den Rest A;
R3 H, (C₁-C₆)-Alkyl, COO(C₁-C₆)-Alkyl, SO₂(C₁-C₆)-Alkyl, (C₁-C₆)-Alkyl-Phenyl, Phenyl, SO₂-Phenyl, wobei der Phenylring jeweils bis zu zweifach mit F substituiert sein kann;
R4 H, (C₁-C₆)-Alkyl, COO(C₁-C₆)-Alkyl, SO₂-(C₁-C₆)-Alkyl, SO₂-Piperidinyl, SO₂-Piperazinyl, (C₁-C₆)-Alkyl-Phenyl, wobei der Phenylring, der Piperidinylring und der Piperazinylring bis zu zweifach mit F oder (C₁-C₆)-Alkyl, substituiert sein kann;
A einen heterozyklischen Rest der Formel 2a oder 2b; 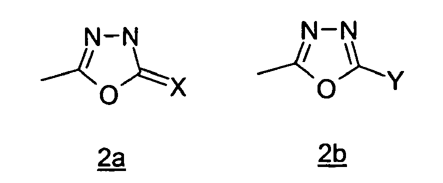
X O oder NH;
Y OH oder NH₂;
sowie deren physiologisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und mindestens einen weiteren Wirkstoff.

6. Arzneimittel, gemäß Anspruch 5, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

7. Verwendung der Verbindungen gemäß einem oder mehreren der Ansprüche 1 bis 3 zur Herstellung eines Medikamentes zur Blutzuckersenkung.

8. Verwendung der Verbindungen gemäß einem oder mehreren der Ansprüche 1 bis 3 zur Herstellung eines Medikamentes zur Behandlung des Typ II Diabetes.

9. Verwendung der Verbindungen gemäß einem oder mehreren der Ansprüche 1 bis 3 zur Herstellung eines Medikamentes zur Behandlung von Lipid- und Kohlenhydratstoffwechselstörungen.

10. Verwendung der Verbindungen gemäß einem oder mehreren der Ansprüche 1 bis 3 zur Herstellung eines Medikaments zur Behandlung arteriosklerotischer Erscheinungen.

11. Verwendung der Verbindungen gemäß einem oder mehreren der Ansprüche 1 bis 3 zur Herstellung eines Medikaments zur Behandlung von Insulin Resistenz.

12. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

13. Verfahren zur Herstellung der Verbindungen der Formel I, **dadurch gekennzeichnet dass** Harnstoffe der Formel 5 oder 3-Aminothiophenderivate der Formel 6, mit Benzoesäurederivaten der Formel 4, 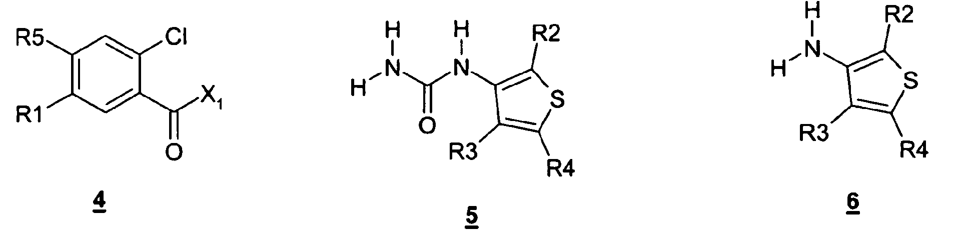 worin R1 bis R5 die in Anspruch 1 angegebenen Bedeutungen haben und X1 gleich Cl oder NCO sein kann, mit Säurechloriden oder Anhydriden bzw. Benzoylisocyanaten, zu Verbindungen der Formel I umgesetzt werden.

14. Verbindungen der Formel I, gemäß Anspruch 1, worin bedeuten 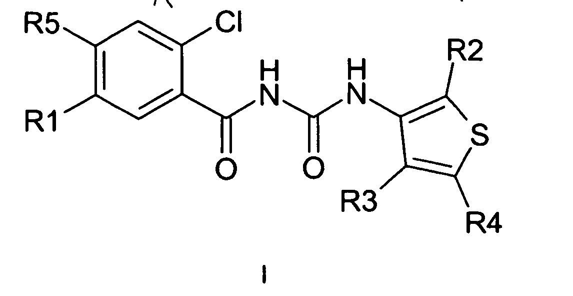
| **R5** | **R1** | **R2** | **R3** | **R4** |
|---|---|---|---|---|
| F | F | COOH | H | 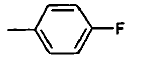 |
| F | F | COOMe | H | 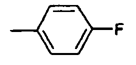 |
| F | F | COOMe | H | Ph |
| F | F | COMe | H | Ph |
| F | F | CONH₂ | H | Ph |
| F | F | CONH₂ | H | 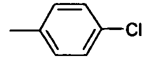 |
| F | F | COOH | H | Ph |
| F | F | CONH₂ | H | 3-Thienyl |
| F | F | CONH₂ | H | 2-Thienyl |
| F | F | CONH₂ | H | 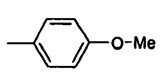 |
| F | F | -CN | H | 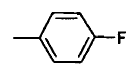 |
| F | F | CONH₂ | H | 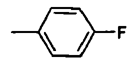 |
| F | F | H | H | Phenyl |
| F | F | 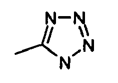 | H | 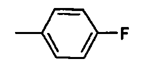 |
| F | H | 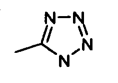 | H | 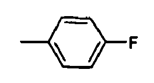 |

## Claims

1. A compound of the formula I 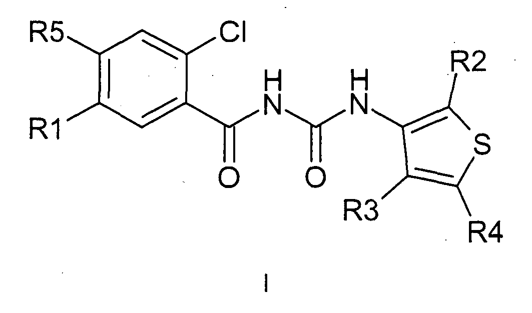 where
R5 is F, Cl or Br;
R1 is H, F, Cl, Br;
R2 is H, F, Cl, Br, (C₁-C₆)-alkyl, CF₃, OCF₃, NO₂, CN, O-(C₁-C₆)-alkyl, CO-(C₁-C₆)-alkyl, COOH, COO(C₁-C₆)-alkyl, CONH₂, CONH(C₁-C₆)-alkyl, CON((C₁-C₆)-alkyl)₂, SO₂-(C₁-C₆)-alkyl, or the A radical;
R3 is H, (C₁-C₆)-alkyl, COO(C₁-C₆)-alkyl, SO₂(C₁-C₆)-alkyl, (C₁-C₆)-alkyl-phenyl, phenyl, SO₂-phenyl, where the phenyl ring may in each case be up to disubstituted by F, Cl, CN, OH, (C₁-C₆)-alkyl, O-(C₁-C₆)-alkyl, CF₃, OCF₃, COOH, COO(C₁-C₆)-alkyl or CONH₂;
R4 is H, (C₁-C₆)-alkyl, COO(C₁-C₆)-alkyl, SO₂-(C₁-C₆)-alkyl, SO₂-piperidinyl, SO₂-Piperazinyl, (C₁-C₆)-alkylphenyl, where the phenyl ring, the piperidinyl ring and the piperazinyl ring may be up to disubstituted by F, Cl, CN, OH, (C₁-C₆)-alkyl, O-(C₁-C₆)-alkyl, CF₃, OCF₃, COOH, COO(C₁-C₆)-alkyl or CONH₂;
A is a heterocyclic radical of the formula 2a, 2b, 2c or 3; 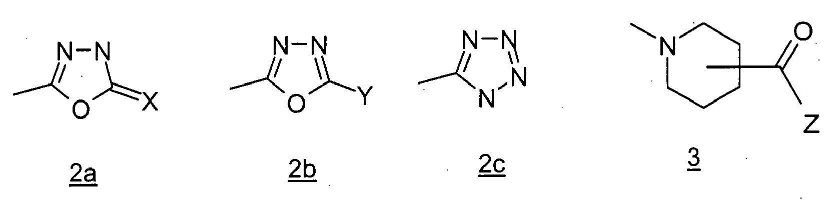
X is O or NH;
Y is OH or NH₂;
Z is OH, O(C₁-C₆)-alkyl, NH₂, NH(C₁-C₆)-alkyl, N((C₁-C₆)-alkyl)₂;
or a compound of the formula I in which 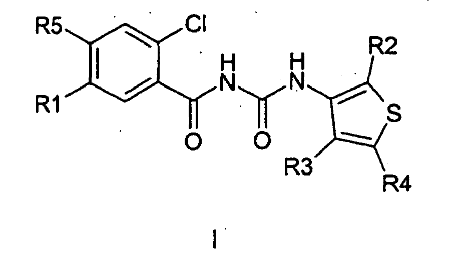
| **R5** | **R1** | **R2** | **R3** | **R4** |
|---|---|---|---|---|
| F | F | COOH | H | 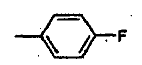 |
| F | F | COOMe | H | 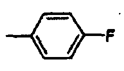 |
| F | F | COOMe | H | Ph |
| F | F | COMe | H | Ph |
| F | F | CONH₂ | H | Ph |
| F | F | CONH₂ | H | 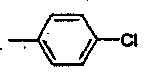 |
| F | F | COOH | H | Ph |
| F | F | CONH₂ | H | 3-thienyl |
| F | F | CONH₂ | H | 2-thienyl |
| F | F | CONH₂ | H | 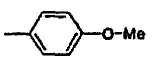 |
| F | F | -CN | H | 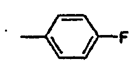 |
| F | F | CONH₂ | H | 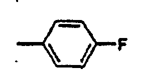 |
| F | F | H | H | phenyl |
| F | F | 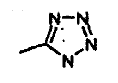 | H | 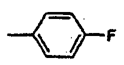 |
| F | H | 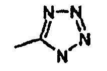 | H | 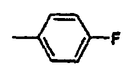 |
and their physiologically tolerated salts.

2. A compound of the formula I as claimed in claim 1, wherein
R5 is F, Cl or Br;
R1 is H, F;
R2 is H, F, Cl, Br, (C₁-C₆)-alkyl, CF₃, OCF₃, NO₂, CN, O-(C₁-C₆)-alkyl, CO(C₁-C₆)-alkyl, COOH, COO(C₁-C₆)-alkyl, CONH₂, CONH(C₁-C₆)-alkyl, CON((C₁-C₆)-alkyl)₂, SO₂-(C₁-C₆)-alkyl, or the A radical;
R3 is H, (C₁-C₆)-alkyl, COO(C₁-C₆)-alkyl, SO₂(C₁-C₆)-alkyl, (C₁-C₆)-alkylphenyl, phenyl, SO₂-phenyl, where the phenyl ring may in each case be up to disubstituted by F or Cl;
R4 is H, (C₁-C₆)-alkyl, COO(C₁-C₆)-alkyl, SO₂-(C₁-C₆)-alkyl, SO₂-piperidinyl, SO₂-Piperazinyl, (C₁-C₆)-alkylphenyl, where the phenyl ring, the piperidinyl ring and the piperazinyl ring may be up to disubstituted by F, Cl, CN, OH, (C₁-C₆)-alkyl, O-(C₁-C₆)-alkyl, CF₃, OCF₃, COOH, COO(C₁-C₆)-alkyl or CONH₂;
A is a heterocyclic radical of the formula 2a, 2b, 2c or 3; 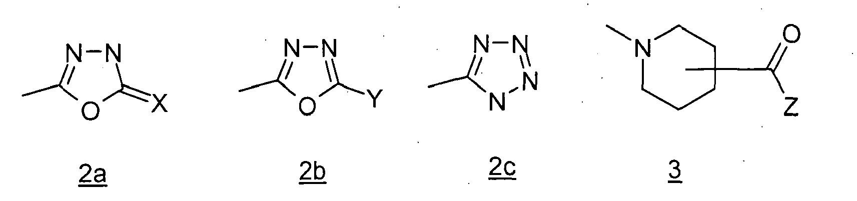
X is O or NH;
Y is OH or NH₂;
Z is OH;
and their physiologically tolerated salts.

3. A compound of the formula I as claimed in claim 1 or 2, wherein
R5 is F;
R1 is F;
R2 is COOH, COO(C₁-C₆)-alkyl, CONH₂, CONH(C₁-C₆)-alkyl, CON((C₁-C₆)-alkyl)₂, or the A radical;
R3 is H, (C₁-C₆)-alkyl, COO(C₁-C₆)-alkyl, SO₂(C₁-C₆)-alkyl, (C₁-C₆)-alkyl-phenyl, phenyl, SO₂-phenyl, where the phenyl ring may in each case be up to disubstituted by F;
R4 is H, (C₁-C₆)-alkyl, COO(C₁-C₆)-alkyl, SO₂-(C₁-C₆)-alkyl, SO₂-piperidinyl, SO₂-piperazinyl, (C₁-C₆)-alkylphenyl, where the phenyl ring, the piperidinyl ring and the piperazinyl ring may be up to disubstituted by F or (C₁-C₆)-alkyl;
A is a heterocyclic radical of the formula 2a or 2b; 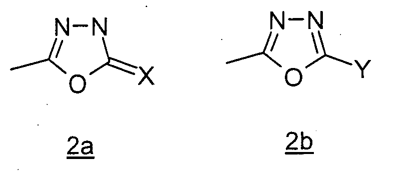
X is O or NH;
Y is OH or NH₂;
and their physiologically tolerated salts.

4. A pharmaceutical comprising one or more of the compounds as claimed in one or more of claims 1 to 3.

5. A pharmaceutical comprising one or more of the compounds as claimed in one or more of claims 1 to 3 and at least one further active ingredient.

6. A pharmaceutical as claimed in claim 5, which comprises, as a further active ingredient, one or more antidiabetics, hypoglycemic active ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients acting on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

7. The use of the compounds as claimed in one or more of claims 1 to 3 for producing a medicament for blood sugar reduction.

8. The use of the compounds as claimed in one or more of claims 1 to 3 for producing a medicament for treating type II diabetes.

9. The use of the compounds as claimed in one or more of claims 1 to 3 for producing a medicament for treating lipid and carbohydrate metabolism disorders.

10. The use of the compounds as claimed in one or more of claims 1 to 3 for producing a medicament for treating arteriosclerotic symptoms.

11. The use of the compounds as claimed in one or more of claims 1 to 3 for producing a medicament for treating insulin resistance.

12. A process for producing a pharmaceutical comprising one or more of the compounds as claimed in one or more of claims 1 to 3, which comprises mixing the active ingredient with a pharmaceutically suitable carrier and converting this mixture into a form suitable for administration.

13. A process for preparing the compounds of the formula I, which comprises reacting ureas of the formula 5 or 3-aminothiophene derivatives of the formula 6 with benzoic acid derivatives of the formula 4 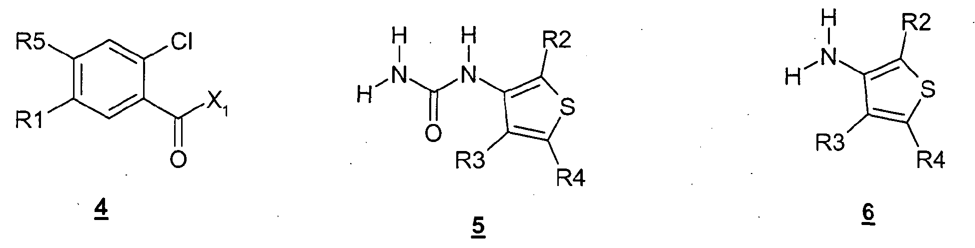 where R1 to R5 are each as defined in claim 1 and X1 may be Cl or NCO, with acid chlorides or anhydrides or benzoyl isocyanates to give compounds of the formula I.

14. A compound of the formula I as claimed in claim 1, in which 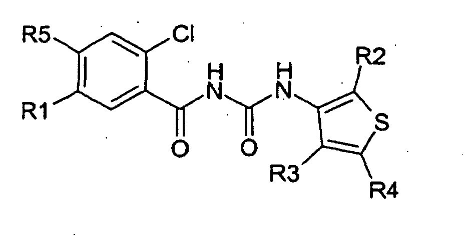
| **R5** | **R1** | **R2** | **R3** | **R4** |
|---|---|---|---|---|
| F | F | COOH | H | 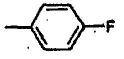 |
| F | F | COOMe | H | 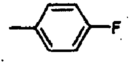 |
| F | F | COOMe | H | Ph |
| F | F | COMe | H | Ph |
| F | F | CONH₂ | H | Ph |
| F | F | CONH₂ | H | 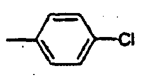 |
| F | F | COOH | H | Ph |
| F | F | CONH₂ | H | 3-thienyl |
| F | F | CONH₂ | H | 2-th ienyl |
| F | F | CONH₂ | H | 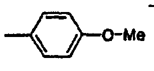 |
| F | F | -CN | H | 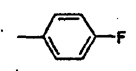 |
| F | F | CONH₂ | H | 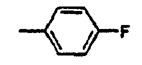 |
| F | F | H | H | phenyl |
| F | F | 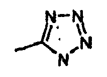 | H | 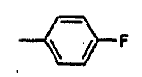 |
| F | H | 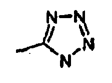 | H | 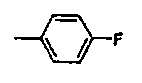 |

## Revendications

1. Composés de formule I, 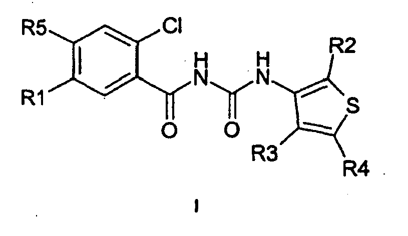 dans laquelle :
R5 représente un atome de fluor, un atome de chlore ou un atome de brome ;
R1 représente un atome d'hydrogène, un atome de fluor, un atome de chlore, un atome de brome ;
R2 représente un atome d'hydrogène, un atome de fluor, un atome de chlore, un atome de brome, un groupe (C₁-C₆)-alkyle, un groupe CF₃, un groupe OCF₃, un groupe NO₂, un groupe CN, un groupe O-(C₁-C₆) -alkyle, un groupe CO(C₁-C₆)-alkyle, un groupe COOH, un groupe COO(C₁-C₆)-alkyle, un groupe CONH₂, un groupe CONH(C₁-C₆)-alkyle, un groupe CON((C₁-C₆)-alkyle)₂, un groupe SO₂-(C₁-C₆)-alkyle, ou le radical A ;
R3 représente un atome d'hydrogène, un groupe (C₁-C₆)-alkyle, un groupe COO(C₁-C₆)-alkyle, un groupe SO₂(C₁-C₆)-alkyle, un groupe (C₁-C₆)-alkylphényle, un groupe phényle, un groupe SO₂-phényle, où le noyau phényle peut, dans chaque cas, être jusqu'à bi-substitué par un atome de fluor, un atome de chlore, un groupe CN, un groupe OH, un groupe (C₁-C₆)-alkyle, un groupe O-(C₁-C₆)-alkyle, un groupe CF₃, un groupe OCF₃, un groupe COOH, un groupe COO(C₁-C₆)-alkyle ou un groupe CONH₂ ;
R4 représente un atome d'hydrogène, un groupe (C₁-C₆)-alkyle, un groupe COO(C₁-C₆)-alkyle, un groupe SO₂- (C₁-C₆)-alkyle, un groupe SO₂-pipéridinyle, un groupe SO₂-pipérazinyle, un groupe (C₁-C₆)-alkylphényle, où le noyau phényle, le noyau pipéridinyle et le noyau pipérazinyle peuvent être jusqu'à bi-substitués par un atome de fluor, un atome de chlore, un groupe CN, un groupe OH, un groupe (C₁-C₆)-alkyle, un groupe O-(C₁-C₆)-alkyle, un groupe CF₃, un groupe OCF₃, un groupe COOH, un groupe COO(C₁-C₆)-alkyle ou un groupe CONH₂ ;
A représente un radical hétérocyclique de formules 2a, 2b, 2c ou 3 : 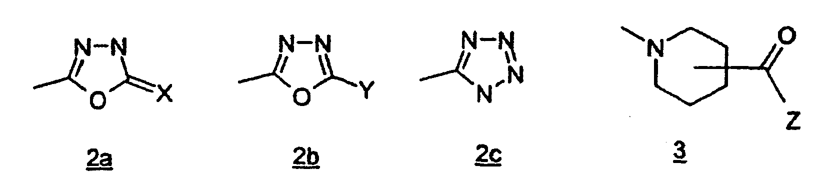
X représente un atome d'oxygène ou un groupe NH ;
Y représente un groupe OH ou un groupe NH₂ ;
Z représente un groupe OH, un groupe O(C₁-C₆)-alkyle, un groupe NH₂, un groupe NH(C₁-C₆)-alkyle, un groupe N((C₁-C₆)-alkyle)₂ ;
ou composés de formule I, dans laquelle : 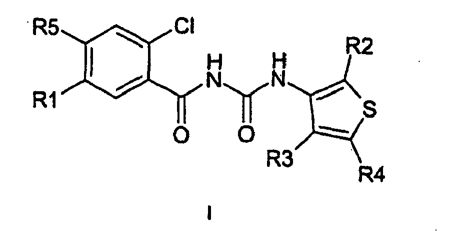
| **R5** | **R1** | **R2** | **R3** | **R4** |
|---|---|---|---|---|
| F | F | COOH | H | 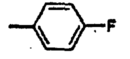 |
| F | F | COOMe | H | 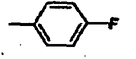 |
| F | F | COOMe | H | Ph |
| F | F | COMe | H | Ph |
| F | F | CONH₂ | H | Ph |
| F | F | CONH₂ | H | 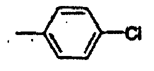 |
| F | F | COOH | H | Ph |
| F | F | CONH₂ | H | 3-thiényle |
| F | F | CONH₂ | H | 2-thiényle |
| F | F | CONH₂ | H | 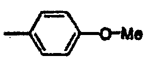 |
| F | F | -CN | H | 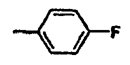 |
| F | F | CONH₂ | H | 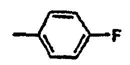 |
| F | F | H | H | phényle |
| F | F | 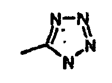 | H | 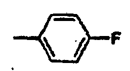 |
| F | H | 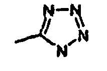 | H | 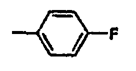 |
ainsi que leurs sels physiologiquement acceptables.

2. Composés de formule I selon la revendication 1, **caractérisés en ce que** :
R5 représente un atome de fluor, un atome de chlore ou un atome de brome ;
R1 représente un atome d'hydrogène, un atome de fluor ;
R2 représente un atome d'hydrogène, un atome de fluor, un atome de chlore, un atome de brome, un groupe (C₁-C₆)-alkyle, un groupe CF₃, un groupe OCF₃, un groupe NO₂, un groupe CN, un groupe O-(C₁-C₆)-alkyle, un groupe CO(C₁-C₆)-alkyle, un groupe COOH, un groupe COO(C₁-C₆)-alkyle, un groupe CONH₂, un groupe CONH(C₁-C₆)-alkyle, un groupe CON ((C₁-C₆-alkyle)₂, un groupe SO₂-(C₁-C₆)-alkyle, ou le radical A ;
R3 représente un atome d'hydrogène, un groupe (C₁-C₆)-alkyle, un groupe COO(C₁-C₆)-alkyle, un groupe SO₂(C₁-C₆)-alkyle, un groupe (C₁-C₆)-alkylphényle, un groupe phényle, un groupe SO₂-phényle, où le noyau phényle peut, dans chaque cas, être jusqu'à bi-substitué par un atome de fluor ou un atome de chlore ;
R4 représente un atome d'hydrogène, un groupe (C₁-C₆)-alkyle, un groupe COO(C₁-C₆)-alkyle, un groupe SO₂- (C₁-C₆)-alkyle, un groupe SO₂-pipéridinyle, un groupe SO₂-pipérazinyle, un groupe (C₁-C₆)-alkylphényle, où le noyau phényle, le noyau pipéridinyle et le noyau pipérazinyle peuvent être jusqu'à bi-substitués par un atome de fluor, un atome de chlore, un groupe CN, un groupe OH, un groupe (C₁-C₆)-alkyle, un groupe O-(C₁-C₆) -alkyle, un groupe CF₃, un groupe OCF₃, un groupe COOH, un groupe COO(C₁-C₆)-alkyle ou un groupe CONH₂ ;
A représente un radical hétérocyclique de formules 2a, 2b, 2c ou 3 : 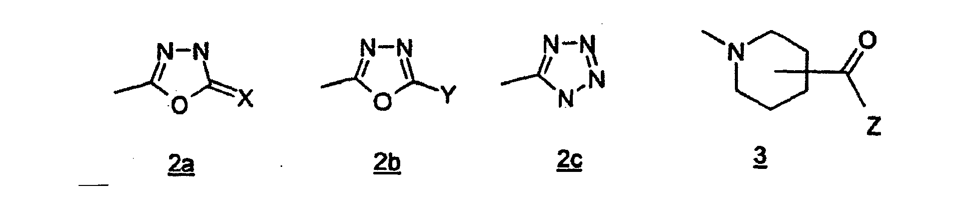
X représente un atome d'oxygène ou un groupe NH ;
Y représente un groupe OH ou un groupe NH₂ ;
Z représente un groupe OH ;
ainsi que leurs sels physiologiquement acceptables.

3. Composés de formule I selon la revendication 1 ou 2, **caractérisés en ce que** :
R5 représente un atome de fluor ;
R1 représente un atome de fluor ;
R2 représente un groupe COOH, un groupe COO(C₁-C₆)-alkyle, un groupe CONH₂, un groupe CONH(C₁-C₆)-alkyle, un groupe CON ((C₁-C₆)-alkyle)₂, ou le radical A ;
R3 représente un atome d'hydrogène, un groupe (C₁-C₆)-alkyle, un groupe COO(C₁-C₆)-alkyle, un groupe SO₂(C₁-C₆)-alkyle, un groupe (C₁-C₆)-alkylphényle, un groupe phényle, un groupe SO₂-phényle, où le noyau phényle peut, dans chaque cas, être jusqu'à bi-substitué par un atome de fluor ;
R4 représente un atome d'hydrogène, un groupe (C₁-C₆)-alkyle, un groupe COO(C₁-C₆)-alkyle, un groupe SO₂- (C₁-C₆)-alkyle, un groupe SO₂-pipéridinyle, un groupe SO₂-pipérazinyle, un groupe (C₁-C₆)-alkylphényle, où le noyau phényle, le noyau pipéridinyle et le noyau pipérazinyle peuvent être jusqu'à bi-substitués par un atome de fluor ou un groupe (C₁-C₆)-alkyle ;
A représente un radical hétérocyclique de formules 2a ou 2b : 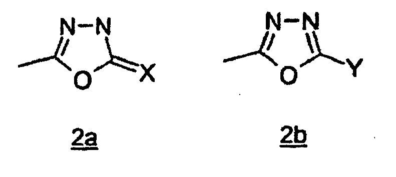
X représente un atome d'oxygène ou un groupe NH ;
Y représente un groupe OH ou un groupe NH₂ ;
ainsi que leurs sels physiologiquement acceptables.

4. Agent pharmaceutique comprenant l'un ou plusieurs des composés selon l'une ou plusieurs des revendications 1 à 3.

5. Agent pharmaceutique comprenant l'un ou plusieurs des composés selon l'une ou plusieurs des revendications 1 à 3 et au moins un ingrédient actif supplémentaire.

6. Agent pharmaceutique selon la revendication 5, **caractérisé en ce qu'**il comprend, en tant qu'ingrédient actif supplémentaire, un ou plusieurs :
agents antidiabétiques, ingrédients actifs hypoglycémiants, inhibiteurs de HMGCoA réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, agents adsorbants polymères de l'acide biliaire, inducteurs de récepteur de LDL, inhibiteurs d'ACAT, agents antioxydants, inhibiteurs de lipoprotéine lipase, inhibiteurs d'ATP citrate lyase, inhibiteurs de squalène synthétase, antagonistes de lipoprotéine(a), inhibiteurs de lipase, insuline, sulfonylurées, biguanides, méglitinides, thiazolidinediones, inhibiteurs de α-glucosidase, ingrédients actifs agissant sur le canal potassique ATP-dépendant des cellules bêta, agonistes de CART, agonistes de NPY, agonistes de MC4, agonistes de l'orexine, agonistes de H3, agonistes de TNF, agonistes de CRF, antagonistes de CRF BP, agonistes de l'urocortine, agonistes de β3, agonistes de la MSH (hormone mélano-stimulante), agonistes de CCK, inhibiteurs du recaptage de la sérotonine, composés mixtes sérotoninergiques et noradrénergiques, agonistes de 5HT, agonistes de la bombésine, antagonistes de la galanine, hormones de croissance, composés libérant une hormone de croissance, agonistes de TRH, modulateurs de la protéine découplante 2 ou 3, agonistes de la leptine, agonistes de DA (bromocriptine, doprexine), inhibiteurs de lipase/amylase, modulateurs de PPAR, modulateurs de RXR ou agonistes de TR-β ou amphétamines.

7. Utilisation des composés selon l'une ou plusieurs des revendications 1 à 3 pour la production d'un médicament destiné à réduire la glycémie.

8. Utilisation des composés selon l'une ou plusieurs des revendications 1 à 3 pour la production d'un médicament destiné au traitement du diabète du type II.

9. Utilisation des composés selon l'une ou plusieurs des revendications 1 à 3 pour la production d'un médicament destiné au traitement de troubles du métabolisme lipidique et glucidique.

10. Utilisation des composés selon l'une ou plusieurs des revendications 1 à 3 pour la production d'un médicament destiné au traitement de symptômes artériosclérotiques.

11. Utilisation des composés selon l'une ou plusieurs des revendications 1 à 3 pour la production d'un médicament destiné au traitement de la résistance à l'insuline.

12. Procédé de préparation d'un agent pharmaceutique comprenant un ou plusieurs des composés selon l'une ou plusieurs des revendications 1 à 3, **caractérisé en ce que** l'ingrédient actif est mélangé avec un support pharmaceutiquement approprié et ce mélange est mis sous une forme appropriée pour l'administration.

13. Procédé de préparation des composés de formule I, **caractérisé en ce que** des urées de formule 5 ou des dérivés 3-aminothiophéniques de formule 6 sont mis à réagir avec des dérivés de l'acide benzoïque de formule 4, 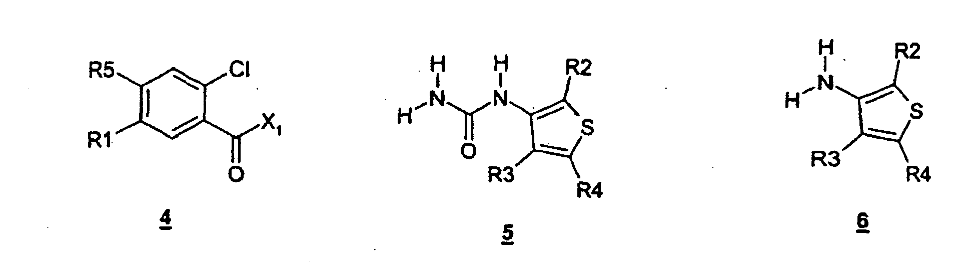 dans laquelle R1 à R5 présentent les significations indiquées dans la revendication 1 et X1 peut représenter un atome de chlore ou un groupe NCO, avec des chlorures d'acide ou des anhydrides, ou des isocyanates de benzoyle, pour donner lieu à des composés de formule I.

14. Composés de formule I selon la revendication 1, dans laquelle : 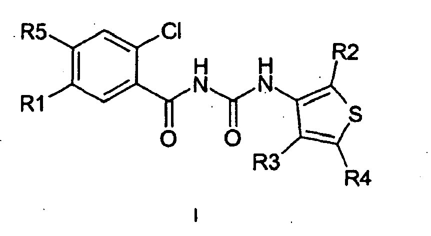
| **R5** | **R1** | **R2** | **R3** | **R4** |
|---|---|---|---|---|
| F | F | COOH | H | 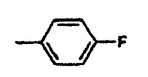 |
| F | F | COOMe | H | 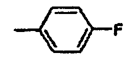 |
| F | F | COOMe | H | Ph |
| F | F | COMe | H | Ph |
| F | F | CONH₂ | H | Ph |
| F | F | CONH₂ | H | 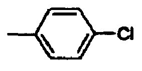 |
| F | F | COOH | H | Ph |
| F | F | CONH₂ | H | 3-thiényle |
| F | F | CONH₂ | H | 2-thiényle |
| F | F | CONH₂ | H | 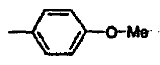 |
| F | F | -CN | H | 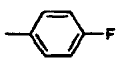 |
| F | F | CONH₂ | H | 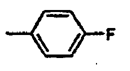 |
| F | F | H | H | phényle |
| F | F | 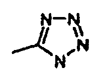 | H | 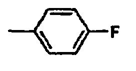 |
| F | H | 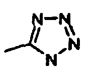 | H | 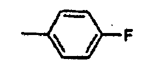 |
